# EUROPEAN PATENT SPECIFICATION

(11) **EP 1 711 126 B1**
(45) Date of publication and mention of the grant of the patent: **18.05.2016**
(21) Application number: 05702119.8
(22) Date of filing: 04.02.2005
(51) Int. Cl.: A61F 2/82

(54) **A DRUG DELIVERY DEVICE**
ARZNEIMITTELABGABEVORRICHTUNG
DISPOSITIF D'ADMINISTRATION DE MEDICAMENTS

(30) Priority: 06.02.2004 GB 0402736
(43) Date of publication of application: 18.10.2006
(73) Proprietor: Vascular Flow Technologies Limited, Dundee DD2 1TY (GB)
(72) Inventor: HOOD, Robert Gordon, Longforgan DD2 5EF (GB); DUFF, Craig McLeod, Dundee,Tayside DD3 9RX (GB)
(74) Representative: Arends, William Gerrit
(86) International application number: PCT/GB2005/000379
(87) International publication number: WO 2005/077305

(56) References cited:
- EP-A- 1 314 406
- WO-A-95/20986
- WO-A-96/28115
- WO-A-03/103540
- WO-A-2004/082533
- DE-A1- 19 516 060

## Description

### Technical Field

The present invention relates to a drug delivery device and, more particularly, an implantable drug delivery device.

### Background Art

It is known in the art to implant intravascular stents into the blood vessels of individuals where there has been stenosis of the blood vessel. Typically, the stent comprises an expandable tube which is introduced into the individual remote from the site of stenosis. The stent is moved into position in the blood vessel of the individual and then, once in place, expanded so as to enlarge the blood vessel and so restore normal blood flow.

WO-A-00/38591, WO-A-03/045280, WO-A-03/045278 and EP 1 314 406 A disclose that an intravascular stent may be provided with a helical formation in its interior so as to induce helical or spiral blood flow in the blood vessel in which it is implanted. The helical formation can be a ridge or a groove. The benefit of introducing helical or spiral blood flow is that turbulent flow and dead regions (which can be a problem in stents without a helical formation) can be eliminated. This, in turn, reduces the likelihood of plaque formation, reduced flow capacity and thromboses in the blood vessel.

Similarly, it is known to implant vascular grafts into a blood vessel to replace a damaged section of blood vessel.

It is often the case that individuals who require the implantation of a stent or graft also require the administration of one or more drugs. Usually this will be due to the implantation of the stent or graft, itself, such as the need to administer a drug to prevent restenosis or to reduce the risk of a thrombus forming in the stent or graft after implantation. However, in some instances, a drug may have to be administered for entirely separate reasons.

It is known in the art to deliver drugs into the blood stream of an individual by introducing the drug into an implant itself, in such a way so as to allow the drug to be steadily released from the implant. This is particularly advantageous if the drug is to be delivered to the area immediately surrounding the implant, for example if the drug is to prevent restenosis. However, the problem with such arrangements is that the surface area of a stent or graft that is actually in contact with the blood of an individual is, in fact, relatively small and because of this the stent or graft has a low drug carrying ability. This is both with respect to the absolute amount of drug that can be carried by the stent and with respect to the rate at which the drug can be delivered to the blood stream of the individual.

For example, DE 195 16 060 A1 which shows all the features of the preamble of claim 1 discloses an implant body for influencing the flow situation in a blood vessel, and a method for the production of an implant body.

WO-A-98/23228 discloses a directional drug delivery stent which includes an elongated or tubular member that has a cavity containing a biologically active agent. The biologically active agent is directionally delivered directly to a desired area of a body lumen, such as the wall of a blood vessel. In one embodiment, the stent is in the shape of a coil or helix in order to permit expansion of the stent. However, the problem with such a directional drug delivery stent is that it still suffers from turbulent flow and/or dead regions in the blood that passes through and around it. Even in the embodiment where the stent is in the shape of a helix, the helix angle is too high to induce helical blood flow and instead tends to exacerbate turbulent blood flow.

WO 2004/082533, which is prior art under Article 54 (3) EPC, discloses a drug delivery stent comprising a helical portion that extends longitudinally and circumferentially.

Accordingly, the present invention seeks to alleviate one or more of the above problems.

### Disclosure of Invention

According to one aspect of the present invention, there is provided a drug delivery device comprising: a drug; and a tubular vascular implant having an inner surface which is blood-contacting and a helical formation located on the blood contacting surface, the drug being releasably associated with the helical formation of the vascular implant. Such a drug delivery device has an improved drug carrying and drug delivery ability because of the increased surface area provided by the helical formation.

The advantage of such a drug delivery device is that it provides a greater area of contact with blood and tissue for release of the drug, when the tubular vascular implant is implanted, compared with a vascular implant lacking a helical formation. Furthermore, the drug delivery device has a single or multiple drug holding capacity many times greater than a vascular implant lacking a helical formation.

The helical formation is capable of inducing helical flow to blood flowing past the helical formation (e.g. flowing through the vascular implant).

Conveniently, the drug is mixed into the material from which the helical formation is made.

Alternatively, the drug is coated onto the surface of the helical formation.

Preferably, the helical formation is made from a polymer, preferably a polymer foam, more preferably polyamide, polyester or polyurethane.

Advantageously, the drug is bound onto the cellular structure of the polymer.

Conveniently, the drug is an anticoagulant, an antiplatelet agent, an angiogenesis inhibitor; a cyclo-oxygenase inhibitor; a gene therapy agent or a mixture of two or more of said drugs.

Preferably, the tubular vascular implant is an intravascular stent, an intravascular stent insert, a vascular graft, or a stent graft.

Advantageously, the tubular vascular implant is a stent and the drug delivery device further comprises a sleeve positioned surrounding and/or within the stent.

Conveniently, the sleeve is made from expanded PTFE.

Preferably, the drug is also releasably associated with the blood-contacting surface of the tubular vascular implant.

Advantageously, one or more further drugs are provided releasably associated with the helical formation and/or the blood-contacting surface of the tubular vascular implant.

Conveniently the helix angle of the helical formation is between 8° and 20°, preferably about 16°.

Preferably, the helical formation comprises at least one fin.

Advantageously the at least one fin has the shape of right-angle triangle in cross-section.

Conveniently, the at least one fin has the shape of an isosceles triangle in cross-section.

Preferably the at least one fin has the shape of a bell in cross-section, more preferably an asymmetric bell.

Alternatively, the helical formation can comprise a groove.

Preferably, the drug is associated with the interior surface of the helical formation or the tubular vascular implant. This ensures that the drug is delivered into the blood stream when the tubular vascular implant is implanted. More preferably the drug is associated only with the interior surface of the helical formation or the tubular vascular implant (and not the exterior surface).

Advantageously, the drug is associated with the exterior surface of the helical formation or the tubular vascular implant. This ensures that the drug is delivered locally to the blood vessel surrounding the tubular vascular implant, when it is implanted. Preferably, the drug is associated only with the exterior surface of the helical formation or the tubular vascular implant (and not the interior surface).

Conveniently, release of the drug is controllable, preferably externally controllable (i.e. externally of the patient's body when the vascular implant is implanted).

Preferably, release of the drug is controllable my means of an adjustable pump.

Alternatively, release of the drug is controllable by means of being associated with a magnetically responsive polymer.

Also herein is a method of delivering a drug to a patient comprising providing a tubular vascular implant comprising a helical formation having the drug releasably associated therewith, wherein the helical formation induces helical flow to blood flowing past the helical formation.

Preferably, the tubular vascular implant comprises the features as described above.

The terms "helix", "helical" and "spiral" as used herein cover the mathematical definition of helix and helical and any combination of mathematical definitions of helical and spiral.

### Brief Description of Drawings

In order that the present invention may be more readily understood and so that further features thereof may be appreciated, embodiments of the invention will now be described, by way of example, with reference to the accompanying drawings in which:-
Figure 1 is a perspective view of a drug delivery device in accordance with one embodiment of the present invention;
Figure 2 is a perspective view of a drug delivery device, having a stent insert, in accordance with another embodiment of the present invention, in a compressed condition;
Figure 3 is a perspective view of the drug delivery device of Figure 2, in an expanded condition;
Figure 4 is a perspective view of the stent insert of the drug delivery device of Figure 2;
Figure 5 is a magnified view of one end of the stent insert of Figure 4;
Figure 6 is a cross-sectional view of the helical formation of another embodiment of the present invention;
Figure 7 is a cross-sectional view of the helical formation of yet another embodiment of the present invention;
Figure 8 is a cross-sectional view of the helical formation of a further embodiment of the present invention; and
Figure 9 is a cross-sectional view of the helical formation of yet another embodiment of the present invention.

### Detailed Description

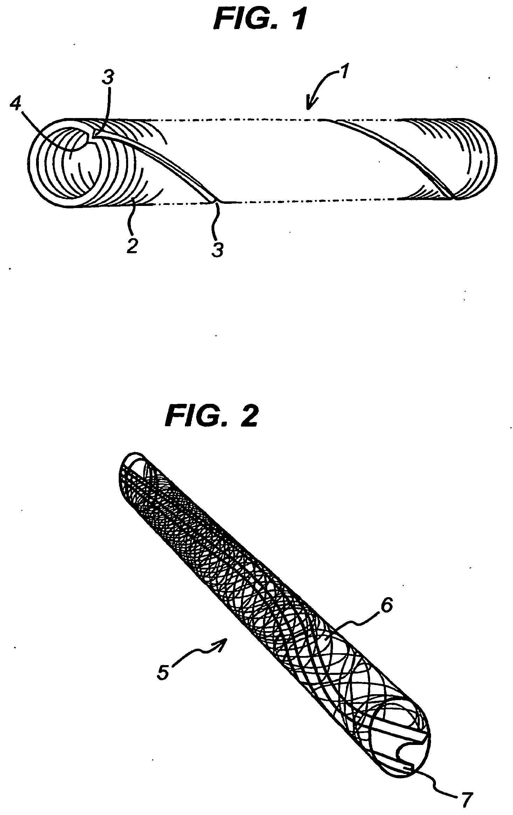

Referring to Figure 1, a drug delivery device 1 comprises a tubular vascular graft 2 into the exterior of which a groove 3 has been pressed. The groove 3 has been pressed in a helical form such that a corresponding helical ridge 4 runs along the length of the interior of the graft 2. Thus the helical ridge 4 forms a helical formation on the graft 2.

In this embodiment, the graft 2 is made from a woven or knitted polymer such as polyamide or polyester. However, the graft could be manufactured from other materials such as polyurethane or expanded polytetrafluoroethane (ePTFE). The graft could also be manufactured from a combination of any of these materials. It is not essential that the graft 2 be made from a polymer, however, and in other embodiments it is made from a ceramic.

In some embodiments, the graft 2 is also coated with a biocompatible material such as a polyurethane. It is preferred that such coatings are polymer coatings which mimic the chemistry of the human body. Most preferred are coatings which are hydrophobic as this provides adhesion and stability. In some embodiments the polymer coatings are crosslinked, which increases their robustness, and in certain embodiments, the coatings are positively or negatively charged.

Mixed into the graft 2 or the coating is an anticoagulant drug such as heparin. In this embodiment, the polymer which forms the graft 2 and the ridge 4 includes the anticoagulant drug. However, in other embodiments, only the polymer which forms the ridge 4 includes the drug.

In use, the graft 2 is implanted into a blood vessel of an individual as is known in the art. Thus a blood vessel of the individual abuts and is attached to either end of the graft 2 (e.g. using sutures) and the blood of the individual flows through the interior of the graft 2. Accordingly, the interior of the graft 2 and the ridge 4 form a blood-contacting surface of the drug delivery device 1. The helical ridge 4 induces a helical spiral flow to the blood.

The anticoagulant drug is mixed into the polymer that forms the graft 2 such that, upon implantation of the graft 2, the drug diffuses from the graft 2 and, in particular, from the ridge 4 into the blood and thus is released into the blood stream of the individual. In some embodiments, the drug is mixed into the polymer so as to be released at a particular time. In other embodiments, the drug is released steadily over a prolonged period of time. The anticoagulant drug lowers the ability of the blood to coagulate and thus reduces the likelihood of a thrombus forming.

It is to be appreciated that the ridge 4 has a considerable surface area in contact with the blood because of its cross-sectional shape. The significant amount of blood which comes into contact with the ridge 4 as it passes through the graft 2 results in the rate of transfer of the drug into the bloodstream being relatively high. Furthermore, a greater quantity of the drug is held adjacent the surface of the graft 2, due to the ridge 4, than is possible with a graft of the same size lacking a helical ridge. Therefore the ridge 4 provides improved drug carrying ability.

In alternative embodiments, the drug is not an anticoagulant drug. For example, in some embodiments, the drug is instead one of the following drugs: an antiplatelet agent; an angiogenesis inhibitor; a cyclo-oxygenase inhibitor; or a gene therapy agent. In further embodiments of the present invention, a mixture of two or more of these drugs is provided.

In the above described embodiment of the present invention, a ridge is provided on the interior of the graft 2. It is to be appreciated that, in other embodiments of the invention, a plurality of helical ridges are provided along the interior of the graft 2, preferably equally spaced about the axis of the graft 2. Furthermore, in alternative embodiments, the ridge is replaced or supplemented with one or more helical grooves on the interior surface of the graft. Each groove works in much the same way as the ridge, causing the blood flow to be helical or spiral. Furthermore, when the graft is implanted, the groove increases the surface area of the graft in contact with blood and thus improves the drug carrying ability of the drug delivery device. Thus the grafts include a helical formation which is either one or more ridges, one or more grooves or a combination of both.

Referring to Figure 2, one embodiment of the present invention is shown. The drug delivery device 5 comprises a wire mesh intravascular stent 6 which is in the form of a compressed tube. Attached to the interior surface of the wire mesh stent 6 is a stent insert 7 made from a polymer foam such as polyurethane. However, it is not essential that the stent be made from a polymer and, in some embodiments, it is, for example made from a ceramic. The insert 7 is elongate, having a broad base 8 and a centrally disposed perpendicular fin 9 such that the stent insert 7 has a bell-shaped cross-section. The insert 7 is attached to the wire mesh stent 6 so that it follows a helical path, about the axis of the stent 6. Thus the insert 7 is a helical formation. A drug is mixed into the polymer foam of the insert 7. In alternative embodiments, the drug is coated onto the surface of the insert 7, again, as described in previous embodiments.

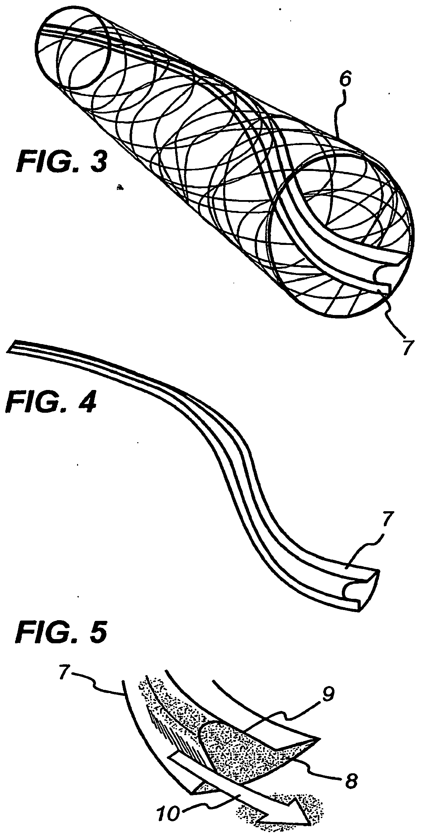

In use, the stent 6 is implanted into a blood vessel of an individual in the usual way. The stent 6 is introduced into a blood vessel of an individual in its compressed form, as is shown in Figure 2. When it is correctly positioned, the stent 6 is opened out into its expanded form as is shown in Figure 3. Thus, a blood vessel of the individual surrounds the stent 6 and the blood of the individual flows through the interior of the stent 6. Accordingly the stent 6 and the insert 7 are in contact with the blood of the individual and the insert 7 imparts a helical flow to the blood. The insert 7 has a considerable surface area in contact with the blood of the individual because of its bell-shaped cross-section and the drug is readily released from the insert 7 (as denoted by the arrow 10 in Figure 5).

Thus the stent insert 7 has a high drug carrying ability, both in terms of the rate at which the drug can be released into the blood and in terms of the absolute amount of drug that is stored adjacent the surface of the insert 7.

In further embodiments of the present invention, the stent insert 7 and the stent 6 form a single, integral stent having a helical formation. In some of these embodiments, the helical formation has a differently shaped cross-section such as a square-shaped or U-shaped cross-section rather than a bell-shaped cross-section as is described in greater detail below. In some of these embodiments, the drug is mixed into or coated on the helical formation, alone, but in other embodiments, the stent, as a whole, is made from or coated in a polymer material in which the drug is mixed or on which the drug is coated. Thus, in these embodiments, the drug is released not only from the helical formation but from the stent, itself.

It is to be appreciated that, in some embodiments, a different drug is mixed into or coated on to the helical formation from that which is mixed into or coated on to the stent, itself. Therefore, these embodiments permit the release of two separate drugs simultaneously. Moreover, the two drugs may be released in different concentrations or over different time periods. It is also to be noted that in some embodiments, the helical formation has more than one drug releasably associated with it

In variants of these embodiments, one or more helical formations (either ridges or grooves) are provided about the interior of the stent, preferably equally spaced about the axis of the stent.

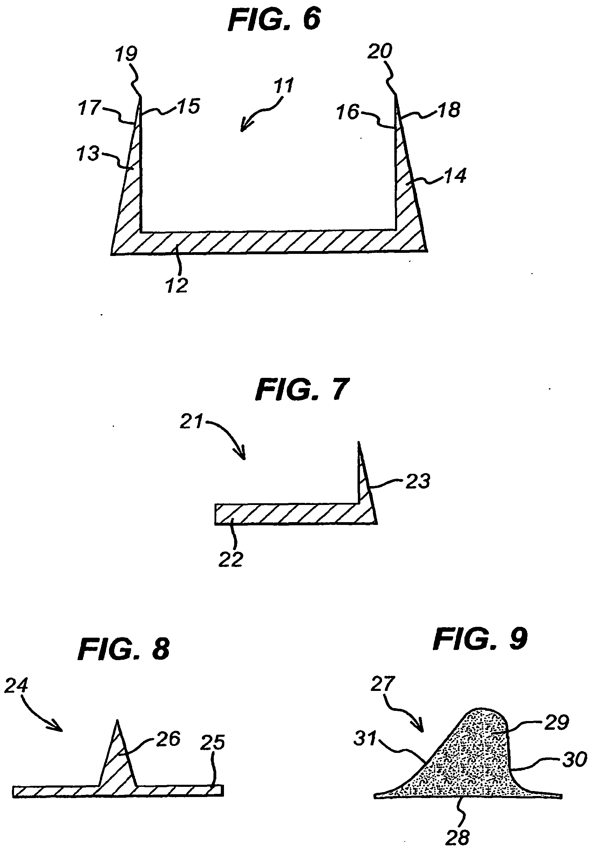

In some embodiments of the present invention, the cross-sectional shape of the helical formation is different from those embodiments previously described. Referring to Figure 6, an embodiment is shown in cross-section in which a helical formation 11 comprises a flat base 12, from either side of which are first and second upwardly extending fins 13, 14. Each of the first and second fins has the cross-section of a right-angled triangle and thus comprises an inner surface 15, 16 which is perpendicular to the base 12 and an outer surface 17, 18 which is at an angle to the base 12 and meets its respective inner surface 15, 16 at an apex 19, 20.

Referring to Figure 7, another embodiment of the present invention Is shown in cross-section. A helical formation 21 having a flat base 22 is provided as in the embodiment shown in Figure 6. However, in this embodiment, only a single upwardly extending fin 23 is provided on one side of the flat base 22. The single fin 23 is identical to the second fin 14 of the embodiment shown in Figure 6.

A further embodiment is shown In Figure 8 in cross-sectional view. A helical formation 24 comprises a flat base 25 from the centre of which is an upwardly extending fin 26. The fin 26 has the cross-section of an isosceles triangle.

Another embodiment of the invention is shown in Figure 9 in cross-sectional view. A helical formation 27 comprises a flat base 28 and a bell-shaped body 29. However, in this embodiment the bell-shaped body 29 is asymmetric, thus the angle of the right side wall 30 with respect to the base 28 is steeper than the angle of the left side wall 31 with respect to the base 28.

In the embodiments of the present invention, because of the helical formations on the interior of the stent, the stent has increased surface area in contact with the blood of the individual which results in the stent having an improved drug carrying ability. Furthermore, the provision of the helical formations on the interior of the stent means that, in embodiments where the drug is provided, mixed into or coated onto only the helical formations, the drug does not come into direct contact with the blood vessel or other organ into which the stent is implanted. Thus the drug is only exposed directly to the flow of blood (or other fluid). This can avoid the delivery of the drug being too concentrated in bodily regions that directly contact the stent. It is also to be noted that the provision of the drug mixed into or coated onto the helical formation gives better release of the drug because the helical formations are positioned more in the flow of the blood than the stent is itself.

In some other embodiments of the present invention, the wire mesh intravascular stent 6 is provided with a sleeve made, for example, from expanded PTFE, that surrounds, or sits inside, the stent, itself. In some of these embodiments, one sleeve is provided around the stent 6 and another sleeve is provided within the stent 6. The effect of the sleeve is to cushion the blood vessel or other surrounding organ of the individual (if the sleeve surrounds the stent 6) or to protect the bloodstream or other fluid from the wire stent 6 (if the sleeve is provided within the stent 6). Where a sleeve is provided within the stent 6, the sleeve, itself, may have the drug mixed in with it, or coated on it, in order to be released as has been described in previous embodiments. Furthermore, in certain embodiments, the insert 7 is integral with the inner sleeve.

Accordingly, it is to be appreciated that the present invention may be implemented with a range of tubular vascular implants having helical formations of which vascular grafts, intravascular stents and stent implants have been described as detailed embodiments herein. In some embodiments, the vascular implant is a stent graft, i.e. a tubular fabric implant. In these embodiments the stent graft is located within a.blood vessel in a patient, and either end of the stent graft is sealed tightly with the interior of the stent graft. The stent graft has a helical formation inside it which induces helical blood flow through the stent graft and with which a drug is releasably associated.

It is to be understood that in the present invention, the helical formation is arranged so as to be capable of inducing helical flow of a fluid, especially blood, that passes through or past the vascular implant. In some embodiments, the helix angle of the helical formation is between 8° and 20°; preferably about 16°, in order to induce helical blood flow.

The advantages that may be achieved by embodiments of the present invention are a reduction in the thickness of the neointimal layer and a decrease in the neointimal area; a reduction in the potential for inflammation; an improvement in the performance (of the delivery of the drug and in blood flow) downstream of the device: the provision of a broad therapeutic window; provision of an environment allowing normal re-endothelialisation and an increase in pharmacokinetics (i.e. an increase in the delivery of a drug when and where it is required).

It is to be appreciated that some embodiments of the invention are particularly effective at delivering the drug downstream of the vascular implant without delivering the drug at all, or only delivering small quantities of the drug, to the blood vessel directly around the vascular implant. In other embodiments, the tubular vascular implant is particularly effective in delivering the drug to the blood vessel directly around the vascular implant but without delivering a drug at all, or only delivering small quantities of the drug, into the blood stream. This is achieved in some embodiments by associating only the exterior surface (i.e. the surface that contacts the blood vessel) of the vascular implant with the drug. For example, in some embodiments only the exterior surface of the vascular implant is coated with the drug. Of course, in some further embodiments, the vascular implant delivers the drug into the bloodstream as well as locally to the blood vessel directly around it.

The processes by which a drug is releasably associated with the tubular vascular implant or its coating will now be described in greater detail.

It is firstly to be understood that there are several different ways that a drug may be releasably associated with the implant. For example, the association of the drug with the vascular implant may be selected from: a drug solution coating; a drug reservoir system with a porous polymer coating or nanoporous ceramic covering; a degradable drug and polymer combination; and a cell membrane lipid bi-layer coating selected from phosphoryl chlorine, phosphoryl ethanolamine or phosphoryl serine. If the tubular vascular implant is a stent then, in some embodiments, the stent is itself, degradable. Furthermore, release of the drug is, in some embodiments, controlled by pharmacokinetics or, in other embodiments, by programmed control.

There are also several different methods by which the drug can be mixed into the tubular vascular implant, itself, or into its coating, depending upon the material from which the vascular implant or coating is made. If the drug is to be mixed into the vascular implant, itself, then this can be done using a drug/polymer mixed solution, to bind the drug into the polymer, or by vacuum loading of the drug into a porous polymer or ceramic.

Alternatively, the drug can be mixed into a polymer that forms the vascular implant by encapsulating it in the polymer. In order to do this, "micro-beads" can be used. In these embodiments, a bead is provided having a large number of channels so that it takes on the form of a sponge. The drug is attached to surfaces on the outside and within the bead, by the drug entering the channels. Nevertheless, the polymer forming the vascular implant is not necessarily a foam and is, for example, in some embodiments polyurethane or another polymer within which the drug is fixed into the microstructure, for release.

In a variation of this embodiment, instead of the drug being mixed into the polymer that forms the tubular vascular implant, the drug is coated onto the interior surface of the vascular implant, in particular the helical formation. In preferred embodiments, the drug is coated by binding it into the cellular structure of the polymer. However, even in this variant, the effect is the same, namely that, when the tubular vascular implant is implanted, the drug is released from the vascular implant, in particular from the helical formation, and into the blood stream of the individual.

If the drug is in the coating of the tubular vascular implant then one method of achieving this is to drip a solution of the polymer coating and drug solution onto the vascular implant. Alternatively, and particularly for temperature-sensitive drugs, the coating, containing the drug, can be cold moulded onto the vascular implant. Another possibility is to pressure mould the coating onto the tubular vascular implant. A further possibility is to coat the vascular implant in two steps. In the first step, an absorbent polymer or ceramic coating is applied to the vascular implant and in the second step, a drug solution is applied to the coating.

It is to be understood that these methods of incorporating drugs into an implant or coating the drug onto an implant are known in the art. As examples of known products which have a drug releasably associated with them, reference may be made to Table 1.

**Table 1**

| **Manufacturer** | **Drug** | **Coating** |
|---|---|---|
| Cordis™ | Rapamune™ | Surmodics ™ Coating Process |
| Boston Scientific™ | Paclitaxel | Polymer based delivery system |
| Guidant ™ | Everolimus (Analogue of Rapamycin) | Resorbable polymer formulation |
| Guidant™ | Everolimus (Analogue of Rapamycin) | Two coat polymer |
| Guidant™ Cook | Paclitaxel | Polymer free coating |
| Medtronic™ Abbott Laboratories™ | ABT-578 Rapamycin | P.C. (phosphorylcholine) |
| Jomed™ | Tacrolimus | Nanoporous ceramic |
| Vascular Architect™ | Nitric-oxide Generator | PTFE coating |
| Cardiotech International™ | Rapamycin | Chronoflex Polyurethane |
| Terumo™ | Angio Tensin 2 Receptor | Polyactic Acid Layer |

In the above described embodiments, the release of the drug from the tubular vascular implant is controlled by the pharmacokinetics of the drug. However, in some other embodiments drug release is actively controlled. For example, in one embodiment the helical formation has a hollow interior and comprises a series of apertures along its length. The drug is contained within the interior and the apertures permit release of the drug. In some variants of this embodiment, a porous membrane replaces the apertures in order to permit release of the drug from the interior. The hollow interior of the helical formation is in fluid communication with a reservoir of the drug and an implantable, remotely adjustable pump. An exemplary pump is the Medtronic™ MiniMed 2007. The pump communicates with an external control device via RF telemetry. Thus operation of the external control device can be used to cause a reduction, an increase or even a cessation of delivery of the drug.

In a further example of actively controlled drug release, the helical formation of some embodiments is not hollow but is instead coated with a drug associated with a magnetically responsive polymer. For instance, in some embodiments, the polymer matrix comprises small magnetic beads. Release of the drug from the polymer is controlled by an external control device which comprises an oscillating bar magnet When the polymer is exposed to the magnetic field of the bar magnet, the drug is released at a greatly increased rate.

## Claims

1. A drug delivery device (1, 5) comprising: a drug; and a tubular vascular implant (2, 6) having an inner surface which is a blood-contacting surface and a helical formation (4, 7, 11, 21, 24, 27) located on the blood-contacting surface, the drug being releasably associated with the helical formation (4, 7, 11, 21, 24, 27) of the vascular implant (2, 6),
**characterised in that** the helical formation (4, 7, 11, 21, 24, 27) is adapted to induce helical flow to blood flowing past the helical formation (4, 7, 11, 21, 24, 27) and wherein the helical formation comprises at least one fin (9, 13, 14, 23, 26, 29) or at least one ridge (4).

2. A drug delivery device according to claim 1 **characterised in that** the drug is mixed into the material from which the helical formation (4, 7, 11,21, 24, 27) is made.

3. A drug delivery device according to claim 1 **characterised in that** the drug is coated onto the surface of the helical formation (4, 7, 11, 21, 24, 27).

4. A drug delivery device according to any one of the preceding claims **characterised in that** the helical formation (4, 7, 11, 21, 24, 27) is made from a polymer, preferably a polymer foam, more preferably polyamide, polyester or polyurethane.

5. A drug delivery device according to claim 4 as dependent on claim 3 **characterised in that** the drug is bound onto the cellular structure of the polymer.

6. A drug delivery device according to any one of the preceding claims **characterised in that** the drug is an anticoagulant, an antiplatelet agent, an angiogenesis inhibitor; a cyclo-oxygenase inhibitor; a gene therapy agent or a mixture of two or more of said drugs.

7. A drug delivery device according to any one of the preceding claims **characterised in that** the vascular implant (2, 6) is an intravascular stent, an intravascular stent insert, a vascular graft, or a stent graft.

8. A drug delivery device according to claim 7 **characterised in that** the vascular implant (2, 6) is a stent and the drug delivery device (1, 5) further comprises a sleeve positioned surrounding and/or within the stent.

9. A drug delivery device according to claim 8 **characterised in that** the sleeve is made from expanded PTFE.

10. A drug delivery device according to any one of the preceding claims **characterised in that** the drug is also releasably associated with the blood-contacting surface of the vascular implant.

11. A drug delivery device according to any one of the preceding claims **characterised in that** one or more further drugs are provided releasably associated with the helical formation (4, 7, 11, 21, 24, 27) and/or the blood-contacting surface of the vascular implant.

12. A drug delivery device according to any one of the preceding claims **characterised in that** the helix angle of the helical formation (4, 7, 11, 21, 24, 27) is between 8° and 20°.

13. A drug delivery device according to any of the preceding claims **characterised in that** the at least one fin (9, 13, 14, 23, 29) has the shape of a right-angle triangle in cross-section.

14. A drug delivery device according to any of claims 1 to 12 **characterised in that** the at least one fin (9, 13, 14, 23, 29) has the shape of an isosceles triangle in cross-section.

15. A drug delivery device according to any of claims 1 to 12 **characterised in that** the at least one fin (9, 13, 14, 23, 29) has the shape of a bell in cross-section, preferably an asymmetric bell.

16. A drug delivery device according to any one of claims 1 to 12 **characterised in that** the helical formation (4, 7, 11, 21, 24, 27) comprises a groove (3).

## Patentansprüche

1. Medikamenten-Verabreichungsvorrichtung (1, 5), umfassend: ein Medikament und ein schlauchförmiges vaskuläres Implantat (2, 6) mit einer inneren Oberfläche, die eine blutkontaktierende Oberfläche ist, und ein spiralförmiges Gebilde (4, 7, 11, 21, 24, 27), das auf der blutkontaktierenden Oberfläche angeordnet ist, wobei das Medikament lösbar mit dem spiralförmigen Gebilde (4, 7, 11, 21, 24, 27) des vaskulären Implantats (2, 6) verbunden ist,
**dadurch gekennzeichnet, dass** das spiralförmige Gebilde (4, 7, 11, 21, 24, 27) ausgelegt ist, um einen spiralförmigen Fluss in das Blut einzuleiten, das an dem spiralförmigen Gebilde (4, 7, 11, 21, 24, 27) vorbeifließt, und wobei das spiralförmige Gebilde mindestens eine Lamelle (9, 13, 14, 23, 26, 29) oder mindestens eine Rippe (4) umfasst.

2. Medikamenten-Verabreichungsvornchtung nach Anspruch 1, **dadurch gekennzeichnet, dass** das Medikament dem Material beigemischt wird, aus dem das spiralförmige Gebilde (4, 7, 11, 21, 24, 27) besteht.

3. Medikamenten-Verabreichungsvornchtung nach Anspruch 1, **dadurch gekennzeichnet, dass** das Medikament auf die Oberfläche des spiralförmigen Gebildes (4, 7, 11, 21, 24, 27) aufgetragen wird.

4. Medikamenten-Verabreichungsvorrichtung nach einem der vorhergehenden Ansprüche, **dadurch gekennzeichnet, dass** das spiralförmige Gebilde (4, 7, 11, 21, 24, 27) aus einem Polymer, bevorzugt aus einem Polymerschaum, stärker bevorzugt aus Polyamid, Polyester oder Polyurethan besteht.

5. Medikamenten-Verabreichungsvorrichtung nach Anspruch 4, sofern von Anspruch 3 abhängig, **dadurch gekennzeichnet, dass** das Medikament an die Zellstruktur des Polymers gebunden ist.

6. Medikamenten-Verabreichungsvornchtung nach einem der vorhergehenden Ansprüche, **dadurch gekennzeichnet, dass** das Medikament ein Gerinnungshemmer, ein Thrombozytenaggregationshemmer, ein Angiogenesehemmer, ein Cyclooxygenasehemmer, ein Gentherapiemittel oder eine Mischung von zwei oder mehreren dieser Medikamente ist.

7. Medikamenten-Verabreichungsvorrichtung nach einem der vorhergehenden Ansprüche, **dadurch gekennzeichnet, dass** das vaskuläre Implantat (2, 6) ein intravaskulärer Stent, ein intravaskulärer Stent-Einsatz, ein vaskuläres Transplantat oder ein Stent-Transplantat ist.

8. Medikamenten-Verabreichungsvorrichtung nach Anspruch 7, **dadurch gekennzeichnet, dass** das vaskuläre Implantat (2, 6) ein Stent ist und die Medikamenten-Verabreichungsvorrichtung (1, 5) ferner eine Hülle umfasst, die um den Stent herum und/oder innerhalb des Stents angeordnet ist.

9. Medikamenten-Verabreichungsvornchtung nach Anspruch 8, **dadurch gekennzeichnet, dass** die Hülle aus expandiertem PTFE besteht.

10. Medikamenten-Verabreichungsvorrichtung nach einem der vorhergehenden Ansprüche, **dadurch gekennzeichnet, dass** das Medikament auch mit der blutkontaktierenden Oberfläche des vaskulären Implantats lösbar verbunden ist.

11. Medikamenten-Verabreichungsvornchtung nach einem der vorhergehenden Ansprüche, **dadurch gekennzeichnet, dass** ein oder mehrere weitere Medikamente in lösbarer Verbindung mit dem spiralförmigen Gebilde (4, 7, 11, 21, 24, 27) und/oder der blutkontaktierenden Oberfläche des vaskulären Implantats bereitgestellt werden.

12. Medikamenten-Verabreichungsvornchtung nach einem der vorhergehenden Ansprüche, **dadurch gekennzeichnet, dass** der Schrägungswinkel des spiralförmigen Gebildes (4, 7, 11, 21, 24, 27) zwischen 8° und 20° liegt.

13. Medikamenten-Verabreichungsvorrichtung nach einem der vorhergehenden Ansprüche, **dadurch gekennzeichnet, dass** die mindestens eine Lamelle (9, 13, 14, 23, 29) im Querschnitt die Form eines rechtwinkligen Dreiecks aufweist.

14. Medikamenten-Verabreichungsvornchtung nach einem der Ansprüche 1 bis 12, **dadurch gekennzeichnet, dass** die mindestens eine Lamelle (9, 13, 14, 23, 29) im Querschnitt die Form eines gleichschenkligen Dreiecks aufweist.

15. Medikamenten-Verabreichungsvornchtung nach einem der Ansprüche 1 bis 12, **dadurch gekennzeichnet, dass** die mindestens eine Lamelle (9, 13, 14, 23, 29) im Querschnitt die Form einer Glocke aufweist, vorzugsweise einer asymmetrischen Glocke.

16. Medikamenten-Verabreichungsvornchtung nach einem der Ansprüche 1 bis 12, **dadurch gekennzeichnet, dass** das spiralförmige Gebilde (4, 7, 11, 21, 24, 27) eine Nut (3) umfasst.

## Revendications

1. Dispositif d'administration de médicament (1, 5) comprenant : un médicament et un implant vasculaire tubulaire (2, 6) ayant une surface interne qui est une surface en contact avec le sang et une formation hélicoïdale (4, 7, 11, 21, 24, 27) située sur la surface en contact avec le sang, le médicament étant associé de manière libérable à la formation hélicoïdale (4, 7, 11, 21, 24, 27) de l'implant vasculaire (2, 6),
**caractérisé en ce que** la formation hélicoïdale (4, 7, 11, 21, 24, 27) est adaptée pour induire un écoulement hélicoïdal au sang s'écoulant au-delà de la formation hélicoïdale (4, 7, 11, 21, 24, 27) et dans lequel la formation hélicoïdale comprend au moins une ailette (9, 13, 14, 23, 26, 29) ou au moins une crête (4).

2. Dispositif d'administration de médicament selon la revendication 1, **caractérisé en ce que** le médicament est mélangé dans le matériau à partir duquel la formation hélicoïdale (4, 7, 11, 21, 24, 27) est réalisée.

3. Dispositif d'administration de médicament selon la revendication 1, **caractérisé en ce que** le médicament est appliqué sur la surface de la formation hélicoïdale (4, 7, 11, 21, 24, 27).

4. Dispositif d'administration de médicament selon l'une quelconque des revendications précédentes, **caractérisé en ce que** la formation hélicoïdale (4, 7, 11, 21, 24, 27) est réalisée à partir d'un polymère, de préférence d'une mousse polymère, encore de préférence de polyamide, de polyester ou de polyuréthane.

5. Dispositif d'administration de médicament selon la revendication 4 lorsqu'elle dépend de la revendication 3, **caractérisé en ce que** le médicament est lié à la structure cellulaire du polymère.

6. Dispositif d'administration de médicament selon l'une quelconque des revendications précédentes, **caractérisé en ce que** le médicament est un anticoagulant, un agent antiplaquettaire, un inhibiteur de l'angiogénèse ; un inhibiteur de la cyclooxygénase ; un agent de thérapie génique ou un mélange de deux ou plus desdits médicaments.

7. Dispositif d'administration de médicament selon l'une quelconque des revendications précédentes, **caractérisé en ce que** l'implant vasculaire (2, 6) est un stent intravasculaire, un insert de stent intravasculaire, un greffon vasculaire ou un greffon d'endoprothèse.

8. Dispositif d'administration de médicament selon la revendication 7, **caractérisé en ce que** l'implant vasculaire (2, 6) est un stent et le dispositif d'administration de médicament (1, 5) comprend en outre un manchon positionné autour de et/ou à l'intérieur du stent.

9. Dispositif d'administration de médicament selon la revendication 8, **caractérisé en ce que** le manchon est réalisé à partir de PTFE expansé.

10. Dispositif d'administration de médicament selon l'une quelconque des revendications précédentes, **caractérisé en ce que** le médicament est également associé de manière libérable avec la surface en contact avec le sang de l'implant vasculaire.

11. Dispositif d'administration de médicament selon l'une quelconque des revendications précédentes, **caractérisé en ce qu'**un ou plusieurs autres médicaments sont prévus en étant associés de manière libérable à la formation hélicoïdale (4, 7, 11, 21, 24, 27) et/ou la surface en contact avec le sang de l'implant vasculaire.

12. Dispositif d'administration de médicament selon l'une quelconque des revendications précédentes, **caractérisé en ce que** l'angle d'hélice de la formation hélicoïdale (4, 7, 11, 21, 24, 27) est compris entre 8° et 20°.

13. Dispositif d'administration de médicament selon l'une quelconque des revendications précédentes, **caractérisé en ce que** la au moins une ailette (9, 13, 14, 23, 29) a la forme d'un triangle rectangle en section transversale.

14. Dispositif d'administration de médicament selon l'une quelconque des revendications 1 à 12, **caractérisé en ce que** la au moins une ailette (9, 13, 14, 23, 29) a la forme d'un triangle isocèle en section transversale.

15. Dispositif d'administration de médicament selon l'une quelconque des revendications 1 à 12, **caractérisé en ce que** la au moins une ailette (9, 13, 14, 23, 29) a la forme d'une cloche en section transversale, de préférence une cloche asymétrique.

16. Dispositif d'administration de médicament selon l'une quelconque des revendications 1 à 12, **caractérisé en ce que** la formation hélicoïdale (4, 7, 11, 21, 24, 27) comprend une rainure (3).
